Europäisches Patentamt

European Patent Office

Office européen des brevets

(19)

(11) Veröffentlichungsnummer: **0 023 556**
**B1**

(12)　**EUROPÄISCHE PATENTSCHRIFT**

(45) Veröffentlichungstag der Patentschrift:
　　 **22.12.82**

(51) Int. Cl.³: **C 07 D 251/32**

(21) Anmeldenummer: **80103394.5**

(22) Anmeldetag: **18.06.80**

(54) **Verfahren zur Herstellung von reiner Cyanursäure.**

(30) Priorität: **19.07.79 DE 2929211**

(43) Veröffentlichungstag der Anmeldung:
　　 **11.02.81 Patentblatt 81/6**

(45) Bekanntmachung des Hinweises auf die Patenterteilung:
　　 **22.12.82 Patentblatt 82/51**

(84) Benannte Vertragsstaaten:
　　 **AT BE CH DE FR GB IT LI NL**

(56) Entgegenhaltungen:
　　 BE-A-704 239
　　 DE-B-1 067 027
　　 US-A-2 943 088
　　 US-A-3 325 493

　　 **Chemical Abstracts vol. 83, no. 13, 29 September 1975 Columbus, Ohio, USA Y. OHATA et al. «Cyanuric acid from melamine» page 569, column 2, abstract No. 114496k**

(73) Patentinhaber: **CHEMIE LINZ AKTIENGESELLSCHAFT, St. Peter-Strasse 25, A-4020 Linz (AT)**

(84) Benannte Vertragsstaaten: **BE CH FR GB IT LI NL AT**

(73) Patentinhaber: **Lentia Gesellschaft mit beschränkter Haftung, Arabellastrasse 4 Postfach 81 05 08, D-8000 München 81 (DE)**

(84) Benannte Vertragsstaaten: **DE**

(72) Erfinder: **Wegleitner, Karlheinz, Dipl.Ing.Dr., Adolf Schärfstrasse 12/I, A-4045 Linz (AT)**
　　 Erfinder: **Krulla, Wilfried, Dipl.Ing., Gferetfeldstrasse 23, A-4050 Traun (AT)**
　　 Erfinder: **Willim, Richard, Denkstrasse 21, A-4020 Linz (AT)**

Verfahren zur Herstellung von reiner Cyanursäure

Die vorliegende Erfindung betrifft ein Verfahren zur Herstellung reiner Cyanursäure aus Roh- bzw. Abfallmelamin, welches insbesondere ammelin- und ammelidhaltig ist, mit Schwefelsäure.

Die meisten Verfahren zur Herstellung von Cyanursäure beruhen auf der Pyrolyse von Harnstoff, wobei ein mehr oder weniger stark verunreinigtes Endprodukt erhalten wird, welches zwecks weiterer Verwendung erst gereinigt werden muss.

Bisher sind nur zwei Verfahren bekannt geworden, welche ausgehend von Melamin bzw. Ammelin oder Ammelid unter Verwendung von Schwefelsäure zu Cyanursäure führen.

So wird in der DE-AS Nr. 1067027 ein Verfahren zur Herstellung von Cyanursäure aus Melamin bzw. Ammelin oder Ammelid beschrieben, gemäss dem als Mineralsäure verdünnte, mindestens 5%ige, bevorzugt 10-20%ige Schwefelsäure, in einem Überschuss bis zu 5% an freier Säure über die auf die vorhandenen Aminogruppen bezogene stöchiometrische Menge hinaus angewendet wird, und die Hydrolyse bei einer Temperatur von mindestens 175°C im geschlossenen Gefäss unter einem Überdruck durchgeführt werden muss. Bevorzugt sind Temperaturen von 180-200°C.

Wie die Nacharbeitung der Beispiele dieser Vorveröffentlichung ergeben hat, ist dieses Verfahren nur dann durchführbar, wenn etwa gemäss Beispiel 1 die Umsetzung mit wesentlich höheren Schwefelsäuremengen als den beanspruchten durchgeführt wird. Das Molverhältnis Melamin zu Schwefelsäure beträgt dort 1 : 3,96. Versucht man aber nach Beispiel 3 Melamin in analoger Weise wie eine Mischung von Ammelin und Ammelid mit 20%iger Schwefelsäure umzusetzen, wobei das molare Verhältnis von Schwefelsäure zu den vorhandenen Aminogruppen dem Schutzbegehren entsprechend 0,52 beträgt, erhält man keine reine sondern auch nach einstündiger Hydrolyse anstelle der angegebenen 10 bis 20 min nur Rohcyanursäure mit einem Gehalt an Verunreinigungen von ca. 15%.

Nach einem wesentlich jüngeren Verfahren (Jap. Kokai 1975, 32193) muss die Hydrolyse von Melamin gleichfalls mit verdünnten Säuren, z.B. Schwefelsäure, durchgeführt werden, wobei die Reaktion zwar unter Normaldruck aber zweistufig erfolgen muss, um reine Cyanursäure zu erhalten. Dabei fallen in der ersten Stufe zunächst Ammelinsalze an, welche in der zweiten Stufe zu Cyanursäure weiterhydrolysiert werden müssen.

Nach der US-PS Nr. 2943088 wird Harnstoff durch Erhitzen in stark verunreinigte Cyanursäure übergeführt, die in einer zweiten Stufe mit Schwefelsäure unter Druck bei erhöhten Temperaturen erst gereinigt werden muss.

Nach der US-PS Nr. 3325493 wird aus melaminhaltigen Mutterlaugen zuerst Melamincyanurat durch Zugabe von Cyanursäure und anschliessende Einstellung eines pH-Wertes zwischen 5,5 und 8 gefällt und der Niederschlag abfiltriert. Dann wird mit Schwefelsäure auch aus der Melaminkomponente bei Temperaturen zwischen 110 bis 250°C Cyanursäure gewonnen. Aus Anspruch 7 ist zu entnehmen, dass 10 bis 50%ige Schwefelsäure verwendet wird und aus Anspruch 7, dass Schwefelsäure in 5 bis 10facher Menge des Nettogewichtes des Niederschlages verwendet werden kann. Zieht man zur Interpretation der Ansprüche die Beschreibung heran, so ist Spalte 3, Zeile 48 ff zu entnehmen, dass Schwefelsäurekonzentrationen von 10-50% zu verwenden sind, wenn nachteilige Nebenreaktionen vermieden werden sollen.

Überraschenderweise wurde nun gefunden, dass man Cyanursäure einer Reinheit von über 99% aus ammelin- und/oder ammelidhaltigem Roh- bzw. Abfallmelamin durch Reaktion von relativ hochkonzentrierter Schwefelsäure ohne nachteilige Zersetzungserscheinungen in einem Einstufenverfahren unter Normaldruck gewinnen kann.

Gegenstand der Erfindung ist somit ein Verfahren zur Herstellung reiner Cyanursäure durch Hydrolyse von Roh- bzw. Abfallmelamin mit Schwefelsäure bei erhöhter Temperatur unter Normaldruck, Zusatz von Wasser unter Abkühlen des Reaktionsgemisches auf Raumtemperatur, Auskristallisieren, Filtrieren, Aufschlämmen und nochmals filtrieren, welches dadurch gekennzeichnet ist, dass für die Hydrolysestufe das Roh- bzw. Abfallmelamin bei Raumtemperatur in eine der Ammoniumbisulfatbildung äquivalente Menge 70-80%iger Schwefelsäure unter Rühren eingetragen wird, worauf das Reaktionsgemisch innerhalb von 1-2 h unter Abdestillieren von mit der Schwefelsäure eingebrachtem Wasser auf Temperaturen von 150-190°C erhitzt wird.

Entgegen der US-PS Nr. 3325493 wird also im erfindungsgemässen Verfahren mit Konzentrationen von 70-80% gearbeitet, ohne dass Nebenreaktionen auftreten. Von diesem entscheidenden Unterschied ganz abgesehen, lässt sich über den Vergleich von Beispielen rechnerisch nachweisen, dass (Beispiel 2 des älteren Verfahrens) mindestens 285,5 mol Schwefelsäure eingesetzt werden, nach dem erfindungsgemässen Verfahren 200,6 mol, das ist die zur Ammoniumbisulfatbildung erforderliche Menge. (Das erfindungsgemäss erhaltene Endprodukt ist mit einem Gehalt von weniger als 0,3% Ammelid optimal rein.)

Bevorzugt sind dabei Schwefelsäurekonzentrationen zwischen 70 und 75% und Reaktionstemperaturen von 160 bis 190°C. Was die erfindungsgemässen Schwefelsäurekonzentrationen betrifft, ist zu sagen, dass bei höheren Konzentrationen als etwa 80% die Gefahr besteht, dass die Reaktion ohne optimal wirksame Kühlung zu heftig wird, und als Folge eine Zersetzung des Triazinringes eintreten kann. Bei Konzentrationen unter 70% wird die Reaktionsdauer, die bei den erfindungsgemässen Reaktionstemperaturen im allgemeinen 3 bis 5 h beträgt, in nachteiliger Weise verlängert. Überraschend ist jedenfalls, dass im Gegensatz zu den bisher bekannten Verfahren nicht von verdünnter Schwefelsäure bis etwa 20% ausgegangen werden muss, um ein reines Produkt zu erhalten, sondern dass relativ hochkonzentrierte Säure bei Normaldruck in einem Einstufenverfahren eingesetzt werden kann, ohne dass die Ausbeute durch Zersetzung des Ausgangs- bzw. Endproduktes zu Ammoniak und Kohlendioxyd leidet. Überschreitungen wie Unterschreitungen des erfindungsgemässen Temperaturbereiches haben für die Ausbeute bzw. Umsetzungsdauer den gleichen Effekt wie ein Über- bzw. Unterschreiten der erfindungsgemässen Schwefelsäurekonzentrationen. Die optimale Reaktionsdauer ist eine Folge der Schwefelsäurekonzentration und Reaktionstemperatur und kann von Fall zu Fall leicht bestimmt werden. Das Abdestillieren des mit der Schwefelsäure eingebrachten Wassers innerhalb von 1-2 h ist deshalb erforderlich, weil bei einem Unterschreiten der Reaktionstemperatur die Reaktionszeit unnötig verlängert wird. Das Roh- bzw. Abfallmelamin wird in die Schwefelsäure eingetragen, da die Reaktion stark exotherm verläuft und sich dadurch die Reaktionstemperatur besser steuern lässt.

Nach Beendigung der Reaktion wird, um ein Ausfallen des Ammoniumbisulfates beim Abkühlen zu vermeiden, so viel Wasser zugesetzt, dass das gebildete Ammoniumbisulfat gelöst bleibt. Durch Aufschlämmen der abfiltrierten Cyanursäure und nochmaliges Abfiltrieren lässt sich deren Reinheit steigern.

Cyanursäurederivate finden Verwendung als Desinfektionsmittel, als Zusätze zu PVC-Weichmachern, Vinylpolymeren und Epoxidharzen.

Die nachstehenden Beispiele sollen das erfindungsgemässe Verfahren näher erläutern.

*Beispiel 1:*

In einem Glaskolben mit Rückflusskühler werden 736 g 80%ige Schwefelsäure vorgelegt und 252 g etwa 97%iges Rohmelamin unter Rühren eingetragen. Die Temperatur wird in einer h bis zum Siedepunkt, der bei 160°C liegt, erhöht. Sollte ein zu starker Temperaturanstieg erfolgen, kann mit Pressluft gekühlt werden. Die anfangs klare Lösung wird vier h am Sieden gehalten. Sollte die entstandene Suspension zu dickflüssig werden, werden einige ml Wasser nachgegeben. Nach Beendigung der Hydrolyse werden 810 ml Wasser zugesetzt, es wird auf Raumtemperatur abgekühlt und die Cyanursäure nach dem Auskristallisieren abfiltriert. Sie wird mit Wasser aufgeschlämmt, gerührt, nochmals abfiltriert und gewaschen. Man erhält 243 g Cyanursäure (94,2% bezogen auf das eingesetzte Rohmelamin) mit einem Ammelidgehalt von weniger als 0,3%.

Sollte das Filtrat frei von Cyanursäure sein, werden nach Beendigung der Hydrolyse 1250 ml Wasser zugesetzt. Unter Kühlen wird NH₃ bis pH 6 eingeleitet. Die Cyanursäure wird nach Abkühlung auf Raumtemperatur abfiltriert und wie oben angegeben gewaschen, man erhält 245 g Cyanursäure (95,0%) mit einem Ammelidgehalt von weniger als 0,3%. Die Ammoniumsulfatlösung wird zum Sieden erhitzt und mit der stöchimetrischen Menge an Melamin behandelt, wodurch die vorhandene Cyanursäure als Melamincyanurat gefällt wird. Das erhaltene Melamincyanurat kann zur Hydrolyse rückgeführt werden, die Ammoniumsulfatlösung ist praktisch frei von organischen Verunreinigungen. Melamincyanurat ist beispielsweise auch als Flammhemmer verwertbar.

*Beispiel 2:*

In einem Emailkessel werden 333 kg 70%iger Schwefelsäure vorgelegt und 100 kg eines Ammelin und Ammelid enthaltenden Abfallmelamins mit einem Melamingehalt von ca. 95% unter Rühren zugesetzt, wodurch die Temperatur auf ca. 90°C ansteigt. Diese Suspension wird bis zum Siedepunkt bei 132°C erhitzt, anschliessend wird unter Abdestillieren von Wasser in einer h auf 160°C aufgeheizt. Die Suspension wird 4 h unter Sieden am Rückfluss auf 160°C gehalten, danach werden 310 l Wasser zugesetzt, wodurch die Temperatur auf 120°C abfällt. Nach Abkühlung auf Raumtemperatur wird die Cyanursäure abzentrifugiert, ausgewaschen, im Gewichtsverhältnis 1 Teil Cyanursäure: 2 Teilen Wasser aufgeschlämmt, eine h bei Raumtemperatur gerührt, abzentrifugiert und gewaschen. Es werden ca. 96 kg (93,9%) einer Cyanursäure mit einem Ammelidgehalt von weniger als 0,3% erhalten. Die Verunreinigung an Ammoniumbisulfat beträgt etwa 0,03%. Melamin und Ammelin sind nicht nachweisbar.

Erhitzt man die Lösung innerhalb von zwei h auf 190°C statt in einer h auf 160°C, verkürzt sich die Reaktionszeit nach Erreichen des Siedepunktes bei 190°C auf ungefähr eineinhalb h. Zur Verbesserung der Rührbarkeit ist es vorteilhaft, mehrmals etwas Wasser nachzugeben, wobei aber der eingestellte Siedepunkt nicht abgesenkt werden soll.

**Patentansprüche**

1. Verfahren zur Herstellung reiner Cyanursäure durch Hydrolyse von Roh- bzw. Abfallmelamin mit Schwefelsäure bei erhöhter Temperatur unter Normaldruck, Zusatz von Wasser unter Abkühlen des Reaktionsgemisches auf Raumtemperatur, Auskristallisieren, Filtrieren, Aufschlämmen und nochmals Filtrieren, dadurch gekennzeichnet, dass für die Hydrolysestufe das Roh- bzw. Abfallmelamin bei Raumtemperatur in eine der Ammoniumbisulfatbildung äquivalente Menge 70-80%iger Schwefelsäure unter Rühren eingetragen wird, worauf das Reaktionsgemisch innerhalb von 1-2 h unter Abdestillieren von mit der Schwefelsäure eingebrachtem Wasser auf Temperaturen von 150-190°C erhitzt wird.

2. Verfahren nach Anspruch 1, dadurch gekennzeichnet, dass die Schwefelsäurekonzentrationen 70-75% betragen.

3. Verfahren nach den Ansprüchen 1 und 2, dadurch gekennzeichnet, dass die Reaktionstemperatur zwischen 160-190°C beträgt.

## Revendications

1. Procédé pour la production d'acide cyanurique pur par hydrolyse de mélamine brute ou de récupération avec de l'acide sulfurique à haute température à la pression normale, par addition d'eau tout en refroidissant le mélange réactionnel à la température ambiante, par séparation, par cristallisation, par filtration, par empâtage et, à nouveau, par filtration, caractérisé en ce qu'on introduit, pour le stade d'hydrolyse, la mélamine brute ou de récupération, à la température ambiante, dans une quantité d'acide sulfurique à 70-80% équivalente à la formation de bisulfate d'ammonium, tout en agitant, après quoi on chauffe le mélange réactionnel en un laps de temps de 1 à 2 h et tout en éliminant l'eau introduite avec l'acide sulfurique, à des températures de 150-190°C.

2. Procédé suivant la revendication 1, caractérisé en ce que les concentrations de l'acide sulfurique sont de 70-75%.

3. Procédé suivant les revendications 1 et 2, caractérisé en ce que la température de réaction est comprise entre 160 et 190°C.

## Claims

1. Process for the preparation of pure cyanuric acid by hydrolyzing crude or waste melamine with sulfuric acid at elevated temperature under normal pressure, adding water, the reaction mixture being cooled to room temperature, crystallizing out, filtering, suspending the product and filtering again, characterized in that, for the hydrolysis stage, the crude or waste melamine is introduced at room temperature, with stirring, into 70-80% strength sulfuric acid in the equivalent amount for formation of ammonium visulfate, after which the reaction mixture is heated to temperatures of 150-190°C in the course of 1-2 h whilst distilling off the water introduced with the sulfuric acid.

2. Process according to claim 1, characterized in that the sulfuric acid concentrations are 70-75%.

3. Process according to claims 1 and 2, characterized in that the reaction temperature is between 160 and 190°C.